# EUROPEAN PATENT APPLICATION

(11) **EP 4 492 046 A1**
(43) Date of publication of application: **15.01.2025**
(21) Application number: 23767161.5
(22) Date of filing: 08.03.2023
(51) Int. Cl.: G01N 21/77, G01N 21/84, G01N 21/64, G01N 33/483

(54) **SPECIMEN TESTING DEVICE AND SPECIMEN TESTING METHOD**

(30) Priority: 08.03.2022 KR 20220029390
(71) Applicant: Ugenecell Inc., Seoul 05557 (KR); Boditech Med Inc., Chuncheon-si, Gangwon-do 24398 (KR)
(72) Inventor: WON, Byoung Yeon, Seoul 06995 (KR); RYU, Seung Hwan, Chuncheon-si Gangwon-do 24377 (KR); IM, Youn Tae, Chuncheon-si Gangwon-do 24401 (KR); CHOI, Kwang Won, Chuncheon-si Gangwon-do 24209 (KR); KIM, Byeong Chul, Chuncheon-si Gangwon-do 24303 (KR)
(74) Representative: Eisenführ Speiser
(86) International application number: PCT/KR2023/003162
(87) International publication number: WO 2023/172060

(57) **Abstract**

A specimen testing device according to an embodiment of the present invention comprises: a specimen reaction part including a plurality of chambers in which different test reagents are accommodated, respectively, wherein a lysis solution having a specimen mixed therein is supplied to each of the chambers to perform a reaction of the specimen with respect to a test reagent; an optical part which radiates a diagnostic beam to each of the chambers while being sequentially moved along the direction in which the plurality of chambers are arranged, and receives an optical signal from each of the chambers; and a diagnosis part in which a plurality of reaction data for one specimen are obtained on the basis of the optical signal according to the test reagent and the wavelength of the diagnostic beam, and which outputs a test result for the specimen on the basis of the reaction data.

## Description

### [Technical Field]

The present invention relates to a specimen testing device and a specimen testing method, and more specifically, to a specimen testing device and a specimen testing method capable of continuously performing various types of specimen reaction tests on one specimen depending on a test reagent and a wavelength of a diagnostic beam.

### [Background Art]

In general, with the development of medicine and various related technologies, tests are being conducted on substances such as blood cells, nucleic acids, proteins, antigens, and the like, contained in certain biological samples such as blood. After collecting the sample as described above, the presence or absence, proportion, and amount of various substances contained in the sample may be tested by analyzing and observing changes that occur after reacting the collected sample with a predetermined reagent, and information on the presence or absence of a disease, a state of the disease, or the like, may be obtained accordingly.

In the sample test process, it is very important to ensure that the sample and the reagent used to test the sample are not affected by external factors and that the correct amount is used each time in obtaining accurate, reproducible results. During the test process, since samples and reagents may be exposed to the outside, it is necessary to effectively prevent contamination due to exposure of the sample and the reagent and ensure test accuracy by using the correct amount.

In addition, it is also necessary to, after the reaction of the reagent and the sample, ensure that a test for detection and reading/analysis of a reaction product is accurately and quickly performed under one integrated system so that test time and test costs are reduced, thereby reducing steps and costs involved in the overall test.

### [Disclosure]

### [Technical Problem]

The present invention is directed to providing a specimen testing device and a specimen testing method capable of continuously performing various types of specimen reaction tests on one specimen depending on a test reagent and a wavelength of a diagnostic beam.

### [Technical Solution]

A specimen testing device according to one embodiment of the present invention includes a specimen reaction part including a plurality of chambers each accommodating different test reagents so that a lysis solution having a specimen mixed therein is provided to each of the chambers and a reaction of the specimen to the test reagent is performed, an optical part configured to irradiate each chamber with a diagnostic beam and receive an optical signal from each chamber while sequentially moving along an arrangement direction of the plurality of chambers, and a diagnosis part configured to acquire a plurality of pieces of reaction data for the one specimen based on the test reagent and the optical signal depending on a wavelength of the diagnostic beam and output a test result of the specimen based on the reaction data.

In addition, each chamber may be provided with a light source incident portion that laterally protrudes, and the optical part may cause the diagnostic beam to enter the light source incident portion.

In addition, the optical part may include a transfer unit installed in parallel with the specimen reaction part and a plurality of optical units coupled to the transfer unit to irradiate each chamber with the diagnostic beam and acquire the optical signal received for each chamber while sequentially moving along the arrangement direction of the chambers.

In addition, the plurality of optical units may include a first optical unit that emits a first diagnostic beam and a second optical unit that is disposed in parallel with the first optical unit and emits a second diagnostic beam, and the first diagnostic beam and the second diagnostic beam may have different fluorescence wavelengths.

The fluorescence wavelengths may include at least one selected from the group consisting of FAM, HEX, ROX, or Cy5, the FAM fluorescence wavelength may have an emission wavelength within a range of 510 nm to 530 nm, the HEX fluorescence wavelength may have an emission wavelength within a range of 550 nm to 580 nm, the ROX fluorescence wavelength may have an emission wavelength within a range of 595 nm to 635 nm, and the Cy5 fluorescence wavelength may have an emission wavelength within a range of 655 nm to 675 nm.

In addition, the first optical unit may be provided to irradiate each chamber with the first diagnostic beam and acquire the optical signal for the first diagnostic beam for each chamber while sequentially moving along the arrangement direction of the plurality of chambers.

In addition, the second optical unit may be provided to irradiate each chamber with the second diagnostic beam and acquire the optical signal for the second diagnostic beam for each chamber while sequentially moving along the arrangement direction of the plurality of chambers.

In addition, the plurality of optical units may further include a third optical unit that is disposed in parallel with the second optical unit along the arrangement direction of the chambers and emits a third diagnostic beam. In addition, the third diagnostic beam may have a different wavelength from the first diagnostic beam and the second diagnostic beam.

In addition, the third optical unit may be provided to irradiate each chamber with the third diagnostic beam and acquire the optical signal for the third diagnostic beam for each chamber while sequentially moving along the arrangement direction of the plurality of chambers.

In addition, the plurality of optical units may further include a fourth optical unit that is disposed in parallel with the third optical unit in the arrangement direction of the chambers and emits a fourth diagnostic beam. In addition, the fourth diagnostic beam may have a different wavelength from the first diagnostic beam to the third diagnostic beam.

In addition, the fourth optical unit may be provided to irradiate each chamber with the fourth diagnostic beam and acquire the optical signal for the fourth diagnostic beam for each chamber while sequentially moving along the arrangement direction of the plurality of chambers.

In addition, the transfer unit may include a transfer rail installed in parallel with the specimen reaction part in the arrangement direction of the plurality of chambers, a transfer belt installed in parallel with the transfer rail and spaced apart from the specimen reaction part, a drive unit including a pair of drive pulleys installed at both ends of the transfer belt and a drive motor that provides a rotational force to one of the drive pulleys, a transfer member having an upper end coupled to the plurality of optical units and a lower end coupled to the transfer belt, wherein the lower end is coupled to the transfer rail to be movable along the transfer rail when the transfer belt operates, and a chain member installed in parallel with and spaced apart from the transfer belt with the transfer rail interposed therebetween, and coupled to the transfer member to guide movement of the transfer member.

In addition, the specimen reaction part may include a container mounting portion into which a vial container containing a lysis solution mixed with the specimen is inserted, a reagent reaction portion in which the plurality of chambers are arranged in a row and spaced apart from each other, a lysis solution distribution portion inserted into the vial container by passing through a lower end of the vial container to receive the lysis solution and distribute the lysis solution to each chamber, and a gasket member stacked between the lysis solution distribution portion and the reagent reaction portion to cover each chamber, wherein the gasket member is provided so that each guide pin for guiding flow of the lysis solution in the chamber passes therethrough and air is allowed to flow into the chamber.

In addition, the specimen testing device may further include a heating part that operates below the reagent reaction portion to provide heat to each chamber so that each chamber is isothermally maintained at a preset temperature and a heat dissipation part coupled to the heating part to dissipate heat from the heating part.

In addition, the lysis solution distribution portion may be provided with a distribution plate provided to correspond to the reagent reaction portion and having an inflow hole vertically passing therethrough in a center thereof, an inflow portion provided to protrude vertically upward from the distribution plate on the same axis as the inflow hole to be insertable into the vial container, a channel portion provided with a reference channel connected to the inflow hole on a lower surface of the distribution plate and branch channels branching off from the reference channel into a plurality of branches to guide flow of the lysis solution flowing into the inflow hole, a plurality of guide pins provided to protrude vertically downward from the distribution plate at ends of the respective branch channels to correspond to the plurality of chambers, and a plurality of air holes provided to vertically pass through the distribution plate and arranged to correspond to the respective guide pins, and the lysis solution distribution portion may be stacked and coupled to the reagent reaction portion so that each guide pin is inserted into the corresponding chamber and each air hole is connected to the corresponding chamber.

In addition, the guide pin may be provided with a pin groove along a longitudinal direction of the guide pin in one surface connected to the branch channel to guide flow of the lysis solution flowing through the branch channel.

In addition, each air hole may be provided to be spaced apart from an end of the branch channel with the corresponding guide pin interposed therebetween on an opposite side of one surface where the pin groove is provided.

In addition, the plurality of branch channels may branch off from the reference channel to be bilaterally symmetrical with respect to the inflow hole, and may be provided to be curved at a predetermined curvature in a direction away from the inflow hole.

In addition, the distribution plate may be provided with a plurality of air grooves having a diameter larger than a diameter of each air hole and arranged to correspond to the respective air holes, and the specimen testing device may further include a filter member inserted into each air groove and provided with a plurality of filter protrusions covering each air hole.

### [Advantageous Effects]

As discussed above, according to a specimen testing device related to at least one embodiment of the present invention, by continuously performing various types of specimen reaction tests on one specimen depending on a test reagent and a wavelength of a diagnostic beam, it is possible to improve the speed and efficiency of testing the specimen.

In addition, by providing a light source incident portion on one side of a chamber to shorten an incident distance of the diagnostic beam, it is possible to improve the precision of test.

### [Description of Drawings]

FIG. 1 schematically shows a configuration diagram of a specimen testing device according to one embodiment of the present invention.
FIGS. 2 and 3 schematically show a perspective view of the specimen testing device according to one embodiment of the present invention.
FIGS. 4 and 5 are views for describing optical units according to one embodiment of the present invention.
FIG. 6 is a set of views for schematically describing an operating state of an optical part according to one embodiment of the present invention.
FIG. 7 schematically shows a perspective view of a test cartridge according to one embodiment of the present invention.
FIG. 8 schematically shows a cross-sectional view taken along line X-X in FIG. 7.
FIG. 9 schematically shows an exploded perspective view of the test cartridge according to one embodiment of the present invention.
FIG. 10 schematically shows a combined state of a reagent reaction portion, a gasket member, and a lysis solution distribution portion as viewed from the rear in a state in which a container mounting portion is removed, in the test cartridge according to one embodiment of the present invention.
FIG. 11 schematically shows a rear view of the reagent reaction portion according to one embodiment of the present invention.
FIGS. 12 and 13 schematically show a structure of the lysis solution distribution portion according to one embodiment of the present invention.
FIGS. 14 to 16 are views for describing a process in which the lysis solution is provided to each chamber, in one embodiment of the present invention.

### [Modes of the Invention]

Since the present invention may be variously modified and embodied, particular embodiments thereof will be illustrated in the drawings and described.

However, this is not intended to limit the present invention to the specific embodiments, and it should be understood to include all modifications, equivalents, and substitutes included in the spirit and scope of the present invention. In describing the present invention, when it is determined that the detailed description of the related known technology may obscure the subject matter of the present invention, the detailed description thereof will be omitted.

Terms "first,", "second," etc., may be used herein to describe various elements, but the elements should not be limited by the terms. Terms are only used to distinguish one element from another element.

The terms used in the present invention are merely provided to describe specific embodiments, and are not intended to limit the present invention. The singular forms are intended to include the plural forms as well, unless the context clearly indicates otherwise.

In the present invention, it will be understood that terms "include," "have," or the like are intended to specify the presence of features, integers, steps, operations, elements, components, and/or combinations thereof stated in the specification, but do not preclude the possibility of the presence or addition of one or more other features, integers, steps, operations, elements, components, or combinations thereof in advance.

Therefore, configurations shown in embodiments described in the specification are merely the most preferred embodiment of the present invention, and, not intended to fully describe the technical aspects of the present invention, so a variety of other equivalents and modifications could be made thereto at the time of the present invention.

In addition, the drawings attached to the present invention should be understood as being enlarged or reduced for convenience of description.

Hereinafter, a specimen testing device and a specimen testing method of the present invention will be described in detail with reference to the attached drawings. However, the attached drawings are illustrative, and the scope of the specimen testing device and the specimen testing method of the present invention is not limited by the attached drawings.

A specimen testing device 100 according to one embodiment of the present invention is a device that continuously performs various types of specimen reaction tests on one specimen depending on a test reagent 10 and a wavelength of a diagnostic beam. The specimen is collected from the human body and provided to a test cartridge 300 in a state of being mixed with a lysis solution contained in a vial container 200. Here, the lysis solution is a solution for extracting nucleic acids by bursting cells, viruses, or the like.

The specimen testing device 100 according to one embodiment of the present invention includes a specimen reaction part 300 including a plurality of chambers each accommodating different test reagents so that a lysis solution having a specimen mixed therein is provided to each of the chambers and a reaction of the specimen to the test reagent is performed, an optical part 600 configured to irradiate each chamber with a diagnostic beam and receive an optical signal from each chamber while sequentially moving along an arrangement direction of the plurality of chambers, and a diagnosis part 700 configured to acquire a plurality of pieces of reaction data for the one specimen based on the test reagent and the optical signal depending on a wavelength of the diagnostic beam and output a test result of the specimen based on the reaction data.

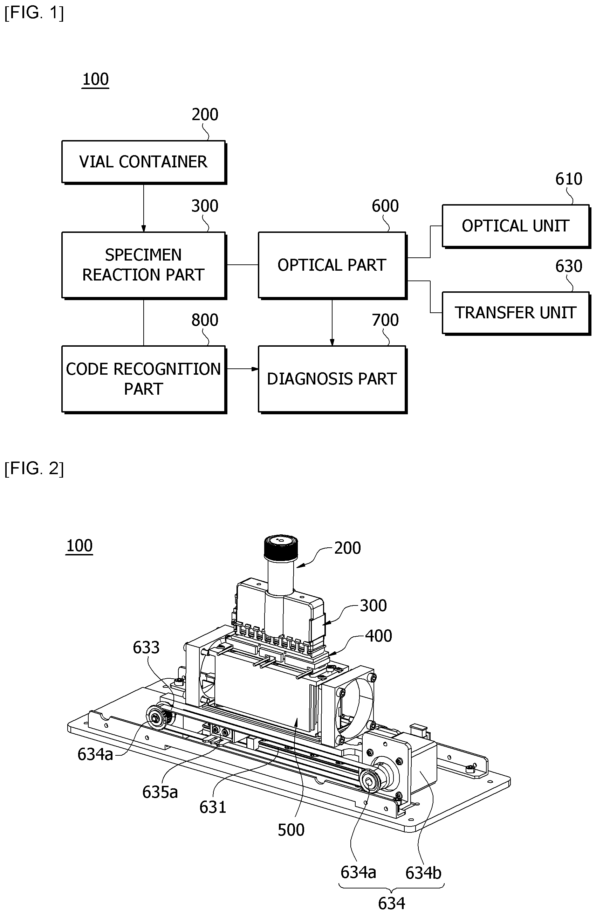

Referring to FIG. 1, the specimen testing device 100 according to one embodiment of the present invention includes the vial container 200, the test cartridge 300, a heating part 400, a heat dissipation part 500, the optical part 600, the diagnosis part 700, and a code recognition part 800.

The test cartridge 300 is provided to perform a reaction of the specimen to the test reagent 10. The vial container 200 is mounted on the test cartridge 300. The structure and function of the test cartridge 300 will be described below.

The vial container 200 accommodates the lysis solution mixed with the collected specimen. The vial container 200 is coupled to the test cartridge 300 and provides the lysis solution to the test cartridge 300. The lysis solution is distributed to each of chambers 311 to 318 provided in the test cartridge 300 and mixed with the test reagent 10 accommodated in the chamber.

Meanwhile, the vial container 200 includes a container body and a container lid 220. The container body includes a solution accommodating portion 211 that accommodates the lysis solution and a punching layer that closes a lower portion of the solution accommodating portion 211. The punching layer is a thin membrane and is provided to be passed through by an inflow portion 332 of the test cartridge 300. In this case, when the punching layer is perforated by the inflow portion 332, the lysis solution in the solution accommodating portion 211 flows into the test cartridge 300.

The container lid 220 is detachably coupled to an upper portion of the container body 210. The container lid 220 is provided with a plurality of lid ventilation holes 221. Air flows into the solution accommodating portion 211 through the lid ventilation holes 211.

That is, the solution accommodating portion 211 is in an atmospheric-pressure state due to the lid ventilation holes 211. Accordingly, when the punching layer is perforated, the lysis solution flows in the direction of gravity even without any special driving device.

As the test reagent 10, various types of reagents may be used depending on the purpose of testing the specimen, and therefore, in the present embodiment, the type of the test reagent 10 is not particularly limited.

The test reagent 10 is accommodated in each of a plurality of chambers 311 to 318 provided in the test cartridge 300. In this case, different types of test reagents 10 are used in each chamber. The test reagent 10 is manufactured in the form of a bead.

The test reagent 10 is made of a test material in the form of a bead. Here, the test material is a material that reacts to a diagnostic beam B with a predetermined wavelength. The diagnostic beam B may have a fluorescence wavelength with which the test cartridge 300 is irradiated by the optical part 600. Each of a plurality of test reagents may be provided to react to a diagnostic beam of a different wavelength.

The heating part 400 heats each of the chambers 311 to 318 to a preset temperature below a reagent reaction portion 310. The heating part 400 operates to maintain the chambers isothermally at a preset temperature.

The heating part 400 contacts remaining areas except the light source incident portions 311a to 318a provided in the chambers and provides heat to the chambers. For example, the heating part 400 may provide heat to three remaining surfaces of the chambers in addition to a surface of the light source incident portions.

The heat dissipation part 500 is coupled to the heating part 400 and dissipates heat from the heating part 400. The heat dissipation part 500 includes a heat dissipation fin 510 and a heat dissipation blowing fan 520. The heat dissipation blowing fan 520 is a fan that provides blowing to the heat dissipation fin 510. The heat dissipation blowing fans 520 may be installed on both sides of the heat dissipation fin 510, respectively.

The optical part 600 is a device for irradiating each of the chambers 311 to 318 of the test cartridge 300 with the diagnostic beam B, receiving an optical signal S from each of the chambers 311 to 3 18, and securing reaction data of the specimen to the test reagent.

In the present embodiment, optical units 610 of the optical part 600 continuously acquire the optical signal S according to the arrangement order of the chambers while sequentially moving in a transfer direction. Here, the transfer direction is parallel to the arrangement direction of the plurality of chambers 311 to 318.

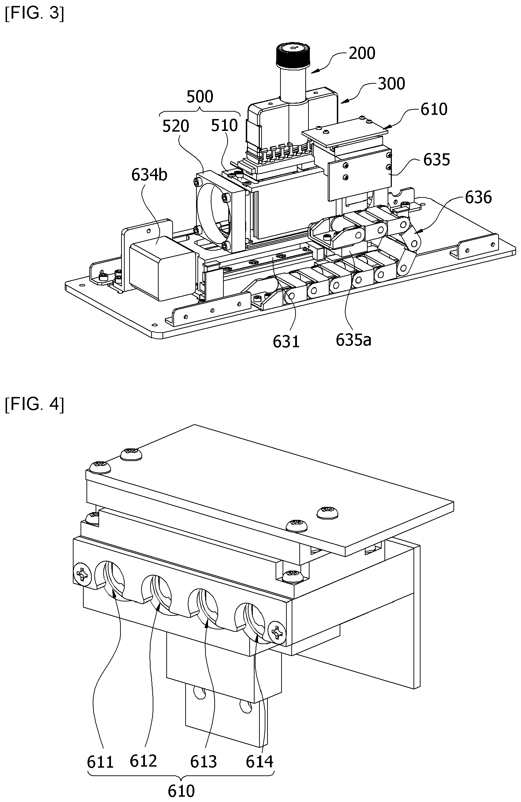

Referring to FIG. 3, the optical part 600 is installed on one side of the test cartridge 300 so that the diagnostic beam B is incident on the light source incident portions 311a to 318a. The optical part 600 includes a plurality of optical units 610 and the transfer unit 630.

The optical units 610 are coupled to the transfer unit 630, and irradiate each of the chambers 311 to 318 with the diagnostic beam and acquire the optical signal S received for each chamber while sequentially moving according to the arrangement order of the chambers. An irradiation direction of the diagnostic beam B is perpendicular to the transfer direction of the optical units.

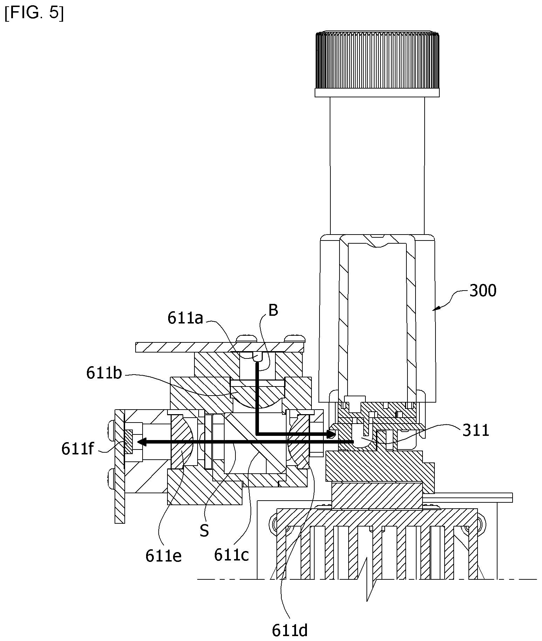

Referring to FIG. 5, the optical unit 610 may include a light source 611a, a first lens 611b, a beam splitter 611c, a second lens 611d, a third lens 611e, and a photodiode 611f.

The diagnostic beam B is incident on the beam splitter 611c from the light source 611a through the first lens 611b, is reflected at a right angle at 90 degrees from the beam splitter 611c, is focused on the second lens 611d, and is incident on the light source incident portions 311a to 318a provided on one side of the chamber. The diagnostic beam B reflected from the chamber passes through the second lens 611d and the beam splitter 611c in a straight line, is focused by the third lens 611e, and is received as the optical signal S by the photodiode 611f

In the present embodiment, for convenience of description, the plurality of optical units 610 are referred to distinguishably as "a first optical unit 611 to a fourth optical unit 614" according to the arrangement order.

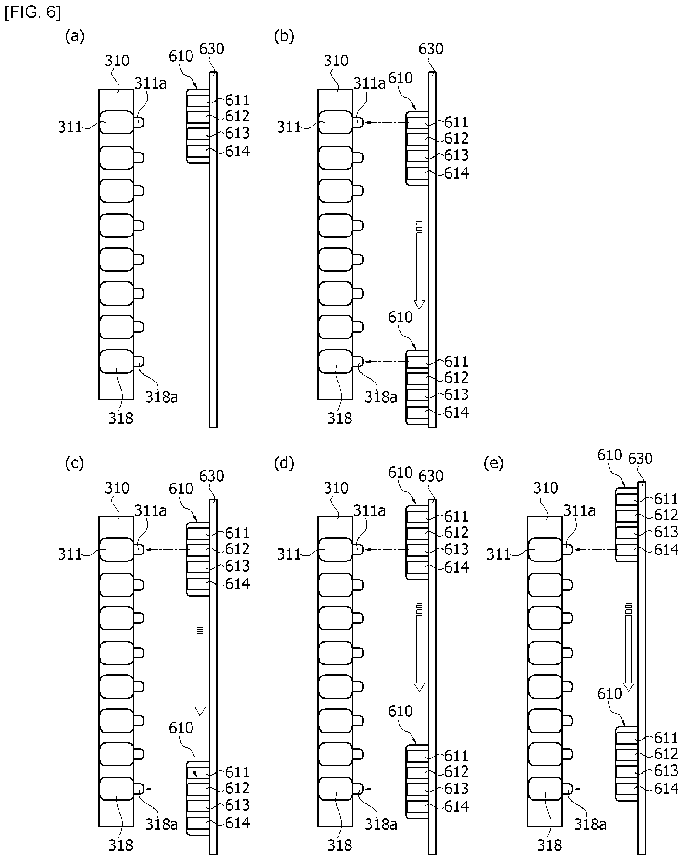

The first optical unit 611 to the fourth optical unit 614 have the same structure except that the optical units emit the diagnostic beams B of different wavelengths. As shown in FIGS. 4 and 6, the first to fourth optical units 611 to 614 are arranged in a row.

The first optical unit 611 is an optical unit 610 that emits a first diagnostic beam B. As shown in FIG. 6B, the position of the first optical unit 611 is adjusted by the transfer unit so that the first diagnostic beam B is vertically incident on the chamber, and the first optical unit 611 irradiates each of the chambers 311 to 318 with the first diagnostic beam B and receives the optical signal S from the photodiode 611f while sequentially moving along the transfer direction according to the arrangement order of the plurality of chambers 311 to 318.

The second optical unit 612 is an optical unit 610 that emits a second diagnostic beam B. The second optical unit 612 is disposed in parallel with the first optical unit 611 in an arrangement direction of the chambers.

As shown in FIG. 6C, the position of the second optical unit 612 is adjusted by the transfer unit so that the second diagnostic beam B is vertically incident on the chamber, and the second optical unit 612 operates to irradiate each of the chambers 311 to 318 with the second diagnostic beam B and receive the optical signal S while sequentially moving along the transfer direction according to the arrangement order of the plurality of chambers 311 to 318.

The third optical unit 613 is an optical unit 610 that emits a third diagnostic beam B. The third optical unit 613 is disposed in parallel with the second optical unit 612 in the arrangement direction of the chamber.

As shown in FIG. 6D, the position of the third optical unit 613 is adjusted by the transfer unit so that the third diagnostic beam B is vertically incident on the chamber, and the third optical unit 613 operates to irradiate each of the chambers 311 to 318 with the third diagnostic beam B and receive the optical signal S while sequentially moving along the transfer direction according to the arrangement order of the plurality of chambers 311 to 318.

The fourth optical unit 614 is an optical unit 610 that emits a fourth diagnostic beam B. The fourth optical unit 614 is disposed in parallel with the third optical unit 613 in the arrangement direction.

As shown in FIG. 6E, the position of the fourth optical unit 614 is adjusted by the transfer unit so that the second diagnostic beam B is vertically incident on the chamber, and the fourth optical unit 614 operates to irradiate each of the chambers 311 to 318 with the fourth diagnostic beam B and receive the optical signal S while sequentially moving along the transfer direction according to the arrangement order of the plurality of chambers 311 to 318.

In the present embodiment, the first to fourth diagnostic beams B have different fluorescence wavelengths. The fluorescence wavelengths include at least one selected from the group consisting of FAM, HEX, ROX, or Cy5.

The FAM fluorescence wavelength has an emission wavelength within a range of 510 nm to 530 nm. The HEX fluorescence wavelength has an emission wavelength within a range of 550 nm to 580 nm. The ROX fluorescence wavelength has an emission wavelength within a range of 595 nm to 635 nm. The Cy5 fluorescence wavelength has an emission wavelength within a range of 655 nm to 675 nm.

The transfer unit 630 is a device that is coupled to a plurality of optical units 610 and transfers the optical units 610.

The transfer unit 630 adjusts the position of any one optical unit to a position at which a first chamber in the arrangement order of the chambers is vertically irradiated with the diagnostic beam B, and transfers any one optical unit at a preset movement interval. Here, the movement interval is an interval between the center of any one chamber and the other chamber adjacent to the chamber.

Referring to FIGS. 2 and 3, the transfer unit 630 includes a transfer rail 631, a transfer belt 633, a drive unit 634, a transfer member 635, and a chain member 636.

The transfer rail 631 is installed in parallel with the test cartridge 300 in the arrangement direction of the chamber. The transfer rail 631 is a rail for guiding the movement of the transfer member 635.

The transfer belt 633 is installed in parallel with the transfer rail 631 and spaced apart from the test cartridge 300. The drive unit 634 is installed on the transfer belt 633.

Referring to FIG. 2, the drive unit 634 includes a pair of drive pulleys 634a and a drive motor 634b. Here, the pair of drive pulleys 634a are rotatably installed at both ends of the transfer belt 633.

One drive pulley 634a of the pair of drive pulleys 634a is coupled to the drive motor 634b. In addition, the one drive pulley 634a transmits a rotational force of the drive motor 634b to the transfer belt 633 when the drive motor 634b rotates. The other drive pulley 634a rotates in idle motion and guides the movement of the transfer belt 633.

The transfer member 635 is coupled to the transfer belt 633. A plurality of optical units 610 are coupled to the upper end of the transfer member 635. In addition, a lower end 635a of the transfer member is coupled to the transfer belt 633 and coupled to the transfer rail 631 to be movable along the transfer rail 631 when the transfer belt 633 operates. Further, the chain member 636 is coupled to the transfer member 635.

The chain member 636 is installed in parallel with and spaced apart from the transfer belt 633 with the transfer rail 631 interposed therebetween, and is coupled to the transfer member 635 to guide the movement of the transfer member 635.

The transfer unit 630 according to the present embodiment adjusts the positions of the optical units 610 as the transfer member 635 moves in a direction of movement of the transfer belt 633 when the drive motor 634b operates.

For example, when the first optical unit 611 operates, the transfer unit 630 adjusts the position of the transfer member 635 so that the first diagnostic beam B of the first optical unit 611 is vertically incident on each of the chambers 311 to 318, and then operates so that the transfer belt 633 moves by a preset movement interval and then is stopped for a preset time.

The movement interval of the transfer belt 633 is a movement interval from one chamber to another adjacent chamber. In addition, a stop time of the transfer belt 633 is a time when the first diagnostic beam B is emitted by the first optical unit 611 and the optical signal S for the first diagnostic beam B is received. The process sequentially proceeds according to the arrangement order of the chambers.

That is, the diagnosis part 700 acquires a plurality of pieces of reaction data for one specimen based on the test reagent 10 and the optical signal S depending on the wavelength of the diagnostic beam B and predicts a test result of the specimen based on the reaction data.

In the diagnosis part 700, the chamber number and the name of the test reagent 10 accommodated in each chamber 311 to 318 are preset according to the arrangement order of the chambers, and the diagnosis part 700 matches the code recognized by the code recognition part 800 with the reaction data according to the chamber number and outputs the test result.

Here, the code recognition part 800 is a device that is installed at a location where a code attached to the test cartridge 300 may be recognized and recognizes a cartridge code 357 attached to a container mounting portion 350.

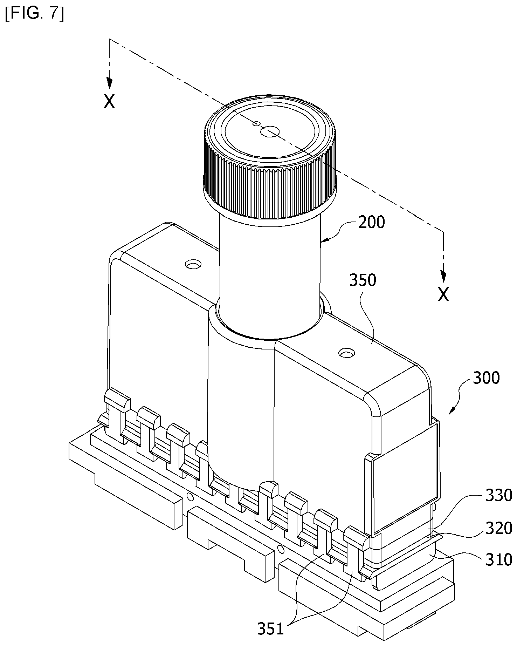

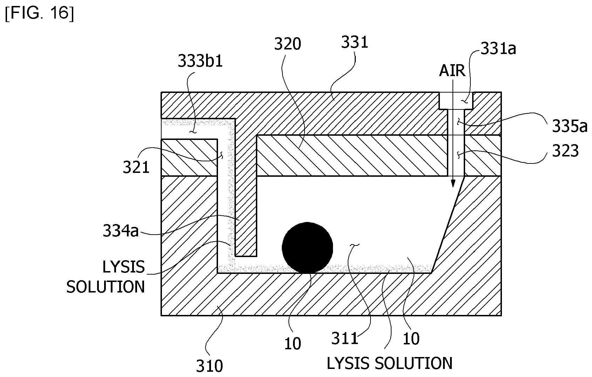

Hereinafter, the test cartridge 300 in which the reaction of the specimen to the test reagent 10 is performed will be described with reference to FIGS. 7 to 16.

Referring to FIGS. 7 to 9, the test cartridge 300 includes the reagent reaction portion 310, a gasket member 320, the lysis solution distribution portion 330, a filter member 340, and a container mounting portion 350. The reagent reaction portion 310, the gasket member 320, the lysis solution distribution portion 330, the filter member 340, and the container mounting portion 350 are sequentially stacked vertically.

The vial container 200 is coupled to the test cartridge 300. Here, the vial container 200 is a container containing a lysis solution mixed with a specimen.

The lysis solution flows down from the vial container 200 through the inflow portion 332 into a channel portion 333 of the lysis solution distribution portion 330 in the direction of gravity when the vial container 200 inserted into the container mounting portion 350 is perforated by the inflow portion 332 of the lysis solution distribution portion 330, flows down into the chambers 311 to 318 along respective guide pins 334a to 334h provided in the channel portion 333, and is stored in each of the chambers 311 to 318.

The reagent reaction portion 310 includes a plurality of chambers 311 to 318. The plurality of chambers 311 to 318 are arranged in a row and spaced apart from each other. Here, a different test reagent 10 is accommodated in each of the chambers 311 to 318. The chamber is a space where the reaction of the specimen to the test reagent 10 is performed.

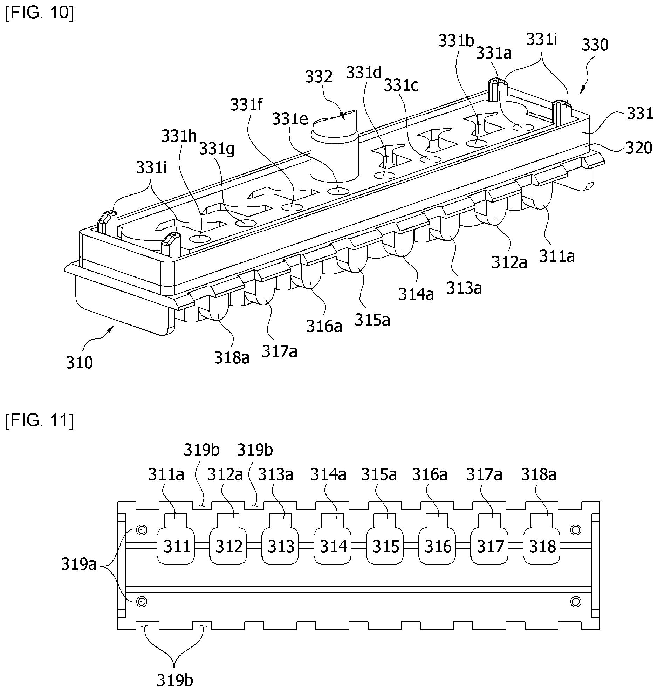

Referring to FIG. 11, the reagent reaction portion 310 is provided with a plurality of upper insertion grooves 319a at an upper edge, and a plurality of upper insertion protrusions of the distribution plate 331 are insertion-coupled into the upper insertion grooves 319a.

The reagent reaction portion 310 is provided with a plurality of side insertion grooves 319b arranged spaced apart along a longitudinal direction on both sides, and the plurality of side insertion grooves 319b are provided so that an arrangement thereof is shifted with respect to the arrangement of a plurality of chambers 311 to 318. Here, the side insertion groove 319b is a groove into which an insertion protrusion 351 of the container mounting portion 350 is coupled.

In the present embodiment, for convenience of description, the plurality of chambers 311 to 318 are referred to distinguishably as "a first chamber 311 to an eighth chamber 318." Here, the first chamber 311 refers to chamber number 1 in the arrangement order. In addition, the eighth chamber 318 refers to chamber number 8 in the arrangement order. However, the number of chambers is variable and is not limited to those described in the specification and drawings.

The chambers 311 to 318 are provided with a laterally protruding light source incident portions 311a to 318a, respectively. In the present embodiment, for convenience of description, the light source incident portions 311a to 318a are referred to distinguishably as "a first light source incident portion 311a to an eighth light source incident portion 318a."

Between the reagent reaction portion 310 and the container mounting portion 350, the gasket member 320, the lysis solution distribution portion 330, and the filter member 340 are sequentially stacked.

The gasket member 320 is disposed above the reagent reaction portion 310 to cover each of the chambers 311 to 318. In this case, the gasket member 320 is preferably installed in the reagent reaction portion 310 so as not to restrict the flow of the lysis solution into each of the chambers 311 to 318.

To this end, a plurality of pin holes 321 and a plurality of gasket air holes 323 are provided in the gasket member 320 in the arrangement direction of the chambers.

Here, the plurality of pin holes 321 are holes through which the respective guide pins 334a to 334h may pass. The pin hole 321 is provided to be larger than a cross-sectional area of the guide pin.

The gasket air hole 323 is a hole through which air passes. The plurality of gasket air holes 323 are provided to correspond to the respective air holes 335a to 335h. The gasket air holes 323 are provided to communicate with the respective chambers 311 to 318. Each gasket air hole 323 is provided coaxially with a corresponding one of the air holes 335a to 335h and a corresponding one of air grooves 331a to 331h to form an air flow path.

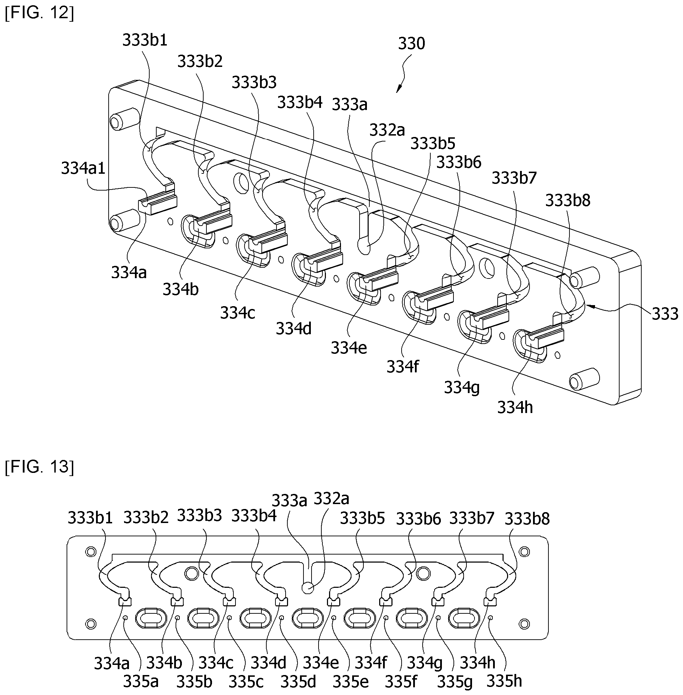

Referring to FIGS. 12 and 13, the lysis solution distribution portion 330 includes the distribution plate 331, the inflow portion 332, the channel portion 333, the plurality of guide pins 334a to 334h, and the plurality of air holes 335a to 335h.

The lysis solution distribution portion 330 is inserted into the vial container 200 through the lower end of the vial container 200, receives the lysis solution, and distributes the lysis solution to each of the chambers 311 to 318. The lysis solution distribution portion 330 is stacked on and coupled to the reagent reaction portion 310 so that each of the guide pins 334a to 334h is inserted into the corresponding one of the chambers 311 to 318 and each of the air holes is connected to the corresponding one of the chambers 311 to 318.

The distribution plate 331 has specifications corresponding to the reagent reaction portion 310. The distribution plate 331 is provided with an inflow hole 332a vertically passing therethrough in the center. An inflow portion 332 is provided on an upper surface of the distribution plate 331, and the channel portion 333 and the plurality of guide pins 334a to 334h are provided on a lower surface thereof.

The distribution plate 331 is provided with a plurality of upper insertion protrusions 331i protruding upward from the distribution plate 331 at an upper surface edge. An upper portion of the distribution plate 331 is insertion-coupled to the container mounting portion 350 by the insertion protrusions 331i.

The distribution plate 331 is provided with a plurality of lower insertion protrusions (not shown) protruding downward from the distribution plate 331 at a lower surface edge. A lower portion of the branch plate is insertion-coupled to the reagent reaction portion 310 by the lower insertion protrusions.

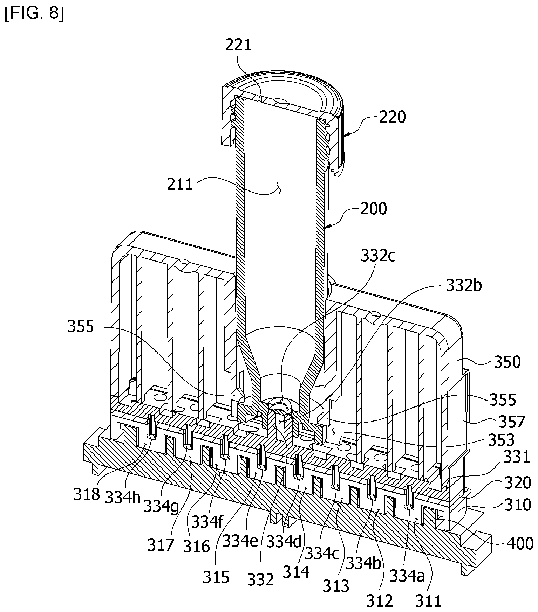

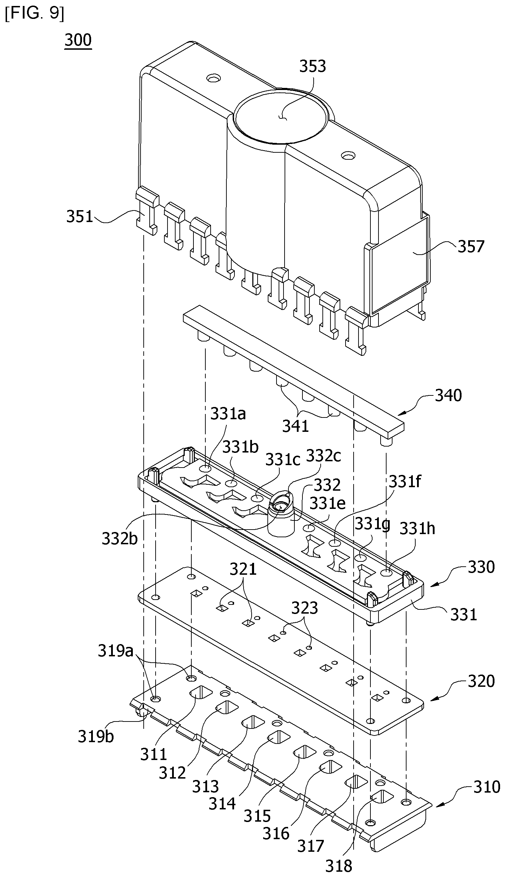

Referring to FIGS. 8 and 9, the inflow portion 332 is provided to protrude vertically upwardly from the distribution plate 331 on the same axis as the inflow hole 332a, and is provided to be inserted into the vial container 200.

The inflow portion 332 has a tip with a sharp upper end. For example, the inflow portion 332 has a pillar shape with a hollow interior.

The inflow portion 332 is provided with an inflow passage 332b and an inflow guide groove 332c. The inflow passage 332b vertically passes through coaxially with the center of the inflow hole 332a. The inflow passage 332b is a passage through which the lysis solution flows from the vial container 200 to the channel portion 333.

The inflow guide groove 332c is provided at the tip portion provided in the inlet 332. The inflow guide groove 332c guides the flow of the lysis solution to the inflow passage 332b. That is, by the inflow guide groove 332c, the lysis solution flows into the inflow passage 332b along the inflow guide groove 332c provided on an inclined surface of the inflow portion in a narrow width, rather than the entire area of the inclined surface.

Referring to FIGS. 12 and 13, the channel portion 333 is provided on the lower surface of the distribution plate 331. The channel portion 333 guides the flow of the lysis solution flowing into the inflow hole 332a.

The channel portion 333 includes a reference channel 333a and a plurality of branch channels 333b1 to 333b8. Here, the reference channel 333a has a groove structure connected to the inflow hole 332a in the lower surface of the distribution plate 331. The plurality of branch channels 333b1 to 333b8 have a groove structure which branches off into several branches from the reference channel 333a.

The plurality of branch channels 333b1 to 333b8 branch off from the reference channel 333a to be bilaterally symmetrical with respect to the inflow hole 332a. The plurality of branch channels 333b1 to 333b8 are provided to be curved with a predetermined curvature in a direction away from the inflow hole 332a. Alternatively, the branch channels 333b1 to 333b8 have an L-shaped structure that is gently bent in a direction away from the inflow hole 332a.

The plurality of branch channels 333b1 to 333b8 are referred to distinguishably as "a first branch channel 333b1 to an eighth branch channel 333b8."

A plurality of guide pins 334a to 334h are provided to protrude vertically downward from the distribution plate at ends of the respective branch channels 333b1 to 333b8 to correspond to the plurality of chambers 311 to 318.

The guide pins 334a to 334h are provided with pin grooves 334a-1 along a longitudinal direction of the guide pins on one surface connected to the branch channels 333b1 to 333b8 to guide the flow of the lysis solution flowing along the respective branch channels 333b1 to 333b8.

In the present embodiment, for convenience of description, the plurality of guide pins 334a to 334h are referred to distinguishably as "a first guide pin 334a to an eighth guide pin 334h." The first guide pin 334a to the eighth guide pin 334h have the same structure.

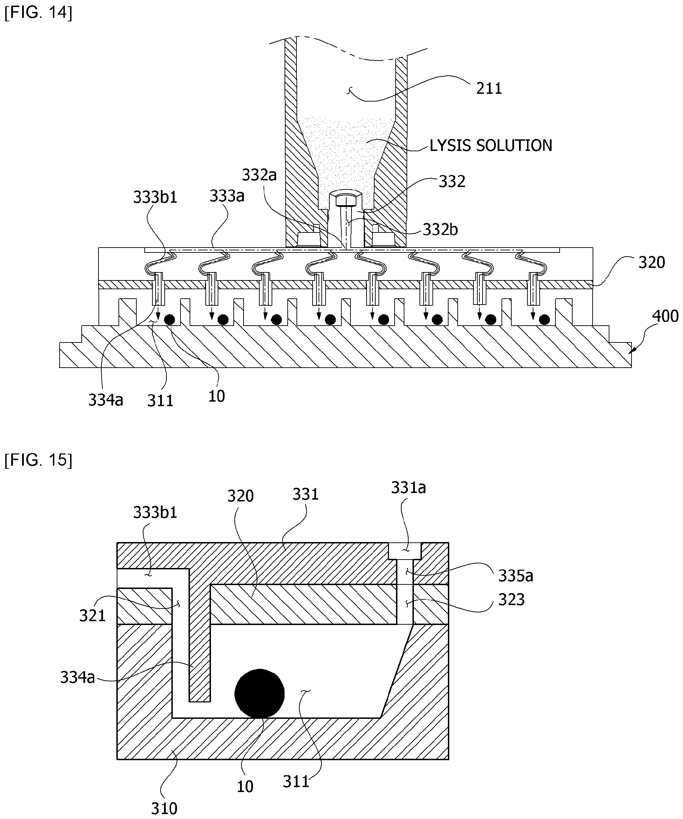

Here, the first guide pin 334a is provided to protrude from the lower surface of the distribution plate 331 at the end of the first branch channel 333b1. Referring to FIGS. 14 to 16, the first guide pin 334a is inserted into the first chamber 311 to guide the flow of the lysis solution into the first chamber 311. In this case, the lysis solution flows down into the first chamber 311 along the pin groove 334a-1 provided in the first guide pin 334a.

Referring to FIG. 14, the second guide pin 334b is provided to protrude from the lower surface of the distribution plate 331 at the end of the second branch channel 333b2. The second guide pin 334b is inserted into the second chamber 312 to guide the flow of the lysis solution into the second chamber 312.

The third guide pin 334c is provided to protrude from the lower surface of the distribution plate 331 at the end of the third branch channel 333b3. The third guide pin 334c is inserted into the third chamber 313 to guide the flow of the lysis solution into the third chamber 313.

The fourth guide pin 334d is provided to protrude from the lower surface of the distribution plate 331 at the end of the fourth branch channel 333b4. The fourth guide pin 334d is inserted into the fourth chamber 314 to guide the flow of the lysis solution into the fourth chamber 314.

The fifth guide pin 334e is provided to protrude from the lower surface of the distribution plate 331 at the end of the fifth branch channel 333b5. The fifth guide pin 334e is inserted into the fifth chamber 315 to guide the flow of the lysis solution into the fifth chamber 315.

The sixth guide pin 334f is provided to protrude from the lower surface of the distribution plate 331 at the end of the sixth branch channel 333b6. The sixth guide pin 334f is inserted into the sixth chamber 316 to guide the flow of the lysis solution into the sixth chamber 316.

The seventh guide pin 334g is provided to protrude from the lower surface of the distribution plate 331 at the end of the seventh branch channel 333b7. The seventh guide pin 334g is inserted into the seventh chamber 317 to guide the flow of the lysis solution into the seventh chamber 317.

The eighth guide pin 334h is provided to protrude from the lower surface of the distribution plate 331 at the end of the eighth branch channel 333b8. The eighth guide pin 334h is inserted into the eighth chamber 318 to guide the flow of the lysis solution into the eighth chamber 318.

The plurality of air holes 335a to 335h are provided to vertically pass through the distribution plate 331, and are arranged to correspond to the respective guide pins 334a to 334h.

The air holes 335a to 335h are provided to be spaced apart from ends of the respective branch channels 333b1 to 333b8 with the corresponding one of the guide pins 334a to 334h interposed therebetween on an opposite side of one surface of the guide pin where the pin groove 334a-1 is provided.

In the present embodiment, for convenience of description, the plurality of air holes are referred to distinguishably as "a first air hole 335a to an eighth air hole 335h."

The first air holes 335a to the eighth air hole 335h are arranged in a row in parallel with the arrangement of the first guide pin 334a to the eighth guide pin 334h.

Referring to FIG. 15, the first air hole 335a is positioned on a straight line with the first guide pin 334a. Similarly, the second air hole 335b to the eighth air hole 335h are respectively positioned on a straight line with the second guide pin 335b to the eighth guide pin 335h.

Here, the first air hole 335a communicates with the first chamber 311 to guide the flow of air into the first chamber 311. The first air hole 335a is positioned on the opposite side of the pin groove 334a-1 and is spaced apart from the first guide pin 334a.

The second air hole 335b communicates with the second chamber 312 to guide the flow of air into the second chamber 312. The third air hole 335c communicates with the third chamber 313 to guide the flow of air into the third chamber 313. The fourth air hole 335d communicates with the fourth chamber 314 to guide the flow of air into the fourth chamber 314.

The fifth air hole 335e communicates with the fifth chamber 315 to guide the flow of air into the fifth chamber 315. The sixth air hole 335f communicates with the sixth chamber 316 to guide the flow of air into the sixth chamber 316.

The seventh air hole 335g communicates with the seventh chamber 317 to guide the flow of air into the seventh chamber 317. The eighth air hole 335h communicates with the eighth chamber 318 to guide the flow of air into the eighth chamber 318.

The distribution plate 331 is provided with a plurality of air grooves 331a to 331h having a diameter larger than a diameter of each air hole and arranged to correspond to the respective air holes.

In the present embodiment, for convenience of description, the plurality of air grooves 331a to 331h are referred to distinguishably as "a first air groove 331a to an eighth air groove 331h."

Referring to FIG. 9, the filter protrusions 341 of the filter member 340 are inserted into the first air groove 331a to the eighth air groove 331h, respectively.

The first air hole 335a is provided in the first air groove 331a. The first air hole 335a is an opening vertically passing through a bottom surface of the first air groove 331a. The first air groove 331a is provided with a diameter larger than a diameter of the first air hole 335a.

Similarly, the second air hole 335b to the eighth air hole 335h are provided in the second air groove 331b to the eighth air groove 331h, respectively.

The first air hole 335a to the eighth air hole 335h are covered by the filter member 340 inserted into the first air groove 331a to the eighth air groove 331h. The filter member 340 allows air to pass therethrough, but filters other foreign substances from entering the air hole. The filter member 340 is provided with a plurality of filter protrusions 341 protruding from one surface.

The plurality of filter protrusions 341 are provided to be insertable into the first air groove 331a to the eighth air groove 331h, respectively. The plurality of filter protrusions 341 are respectively inserted into the first air groove 331a to the eighth air groove 331h to cover the air holes 335a to 335h.

The vial container 200 containing the lysis solution mixed with the specimen is inserted into the container mounting portion 350. The container mounting portion 350 is provided with a container insertion space 353, a plurality of hooks 355, a plurality of insertion protrusions 351, and a cartridge code 357.

Referring to FIG. 8, the container insertion space 353 is a space into which the vial container 200 is inserted.

The plurality of hooks 355 are provided in the container insertion space 353 to protrude inside the container insertion space 353. The hooks 355 are inserted into the vial container 200 to prevent the vial container 200 from being separated from the container insertion space 353.

The cartridge code 375 is a part where a code name of the test cartridge 300 on which the test reagent 10 is processed is written.

The plurality of insertion protrusions 351 are protrusions that protrude vertically downward from the container mounting portion 350. The plurality of insertion protrusions are insertion-coupled to the side insertion grooves 319b of the reagent reaction portion 310. Accordingly, the container mounting portion 350 is coupled to the reagent reaction portion 310. The container mounting portion 350 and the reagent reaction portion 310 are coupled to be vertically stacked.

When the vial container 200 is inserted into the container insertion space 353, the hooks 355 support the side of the vial container 200, allowing the lysis solution in the vial container 200 to flow in the direction of gravity, so that the container mounting portion 350 stably holds the vial container 200.

When the lysis solution flows into the chambers, the container mounting portion 350 stably holds the vial container 200, preventing the vial container 200 from shaking, so that the lysis solution may be evenly distributed in each of the chambers 311 to 318.

Hereinafter, a process of continuously testing a specimen using the present invention will be described.

The vial container 200 is inserted into the container mounting portion 350. The punching layer provided at a lower portion of the vial container 200 is perforated by the inflow portion 332 of the lysis solution distribution portion 330. Accordingly, the lysis solution in the vial container 200 flows into the channel portion 333 via the inflow portion 332. In this case, the flow of the lysis solution proceeds in the direction of gravity.

The lysis solution branches off into the plurality of branch channels 333b1 to 333b8 of the channel portion 333 and flows into the chambers along the pin groove 334a-1 of each of the guide pins 334a to 334h provided at the ends of the respective branch channels 333b1 to 333b8.

For example, the lysis solution flows into the first chamber 311 along the first guide pin 334a of the lysis solution distribution portion 330. The first air hole 335a of the lysis solution distribution portion 330 communicates with the gasket air hole 323 and is connected to the first chamber 311.

As air flows into the first chamber 311 through the first air hole 335a and the gasket air hole 323, the first chamber 311 is affected by gravity. Accordingly, the lysis solution flows down along the first guide pin 334a in the direction of gravity and into the first chamber 311. Here, the lysis solution is in a state of being mixed with the specimen.

In the first chamber 311, the specimen in the lysis solution is mixed with a first test reagent 10 accommodated in the first chamber 311. For specimen reaction between the specimen and the first test reagent 10, the first chamber 311 is isothermally maintained at a temperature suitable for the chemical reaction. The first chamber 311 is heated to a predetermined temperature by the heating part 400.

The first test reagent 10 is accommodated in the first chamber 311. Further, second to eighth test reagents 10 to 10 are accommodated in the second to eighth chambers 312 to 318, respectively. As the first test reagent 10 to the eighth test reagent 10, different types of reagents may be used.

After a preset time has elapsed, the first optical unit 611 sequentially moves along the arrangement direction of the chambers at the movement interval between chambers.

Specifically describing, the position of the first optical unit 611 is adjusted by the transfer unit 630 so that the first diagnostic beam B is vertically incident toward the first light source incident portion 311a of the first chamber 311. Subsequently, the first optical unit 611 causes the first diagnostic beam B to be incident on the first light source incident portion 311a of the first chamber 311 and receives the optical signal S reflected from the first light source incident portion 311a.

Next, the first optical unit 611 is moved by the transfer unit 630 to a position where the first diagnostic beam B is vertically incident on the second light source incident portion 312a of the first chamber 311. A moving distance of the first optical unit 611 is a moving distance between the first chamber 311 and the second chamber 312. The first optical unit 611 provides the first diagnostic beam B to the second light source incident portion 312a and receives the optical signal S reflected from the second light source incident portion 312a.

The process is repeated while the first optical unit 611 sequentially moves from the first chamber 311 toward the eighth chamber 318. Accordingly, the first optical unit acquires eight optical signals S for the first to eighth chambers 311 to 318.

When the operation of the first optical unit 611 is completed, the position of the second optical unit 612 is adjusted by the transfer unit 630 so that the second optical unit 612 irradiates the first light source incident portion 311a of the first chamber 311 with the second diagnostic beam B. The second optical unit 612 also acquires eight optical signals S for the first to eighth chambers 311 to 318 while sequentially moving along the transfer direction. The process also proceeds in the same way for the third optical unit 613 and the fourth optical unit 614.

Through the above process, the optical signal S may be acquired for each wavelength of the diagnostic beam B for the first test reagent 10.

For example, when two optical signals S for the first chamber 311 are acquired by two optical units, that is, the first optical unit 611 and the second optical unit 612, the diagnosis part 700 may calculate the test result for the first test reagent 10 through the two optical signals S depending on the wavelengths of the respective diagnostic beams B, that is, the optical signal S acquired by the first optical unit 611 in the first chamber 311, and the optical signal S acquired by the second optical unit 612 in the first chamber 311.

Alternatively, when three optical signals S for the first chamber 311 are acquired by three optical units, that is, the first to third optical units 611 to 613, the diagnosis part 700 may calculate the test result for the first test reagent 10 through the three optical signals S depending on the wavelengths of the respective diagnostic beams B.

Accordingly, the present invention may continuously irradiate a plurality of test reagents 10 with the diagnostic beams B of different wavelengths, continuously acquire the optical signals S for the respective test reagents 10 depending on the wavelengths of the respective diagnostic beams B, and calculate the test result of the specimen for the respective test reagents 10 from the acquired optical signals S.

Therefore, the present invention uses a specimen collected once to continuously perform several tests in one flow under the same conditions, thereby preventing contamination of the collected specimen and improving the accuracy and speed of the test.

The preferred embodiments of the present invention described above have been disclosed for illustrative purposes, those skilled in the art should be able to make various modifications, changes, and additions within the spirit and scope of the present invention, and such modifications, changes, and additions should be regarded as falling within the scope of the following claims.

### [Industrial Applicability]

According to a specimen testing device related to at least one embodiment of the present invention, by continuously performing various types of specimen reaction tests on one specimen depending on a test reagent and a wavelength of a diagnostic beam, it is possible to improve the speed and efficiency of testing the specimen.

## Claims

1. A specimen testing device comprising:
a specimen reaction part including a plurality of chambers each accommodating different test reagents so that a lysis solution having a specimen mixed therein is provided to each of the chambers and a reaction of the specimen to the test reagent is performed;
an optical part configured to irradiate each chamber with a diagnostic beam and receive an optical signal from each chamber while sequentially moving along an arrangement direction of the plurality of chambers; and
a diagnosis part configured to acquire a plurality of pieces of reaction data for the one specimen based on the test reagent and the optical signal depending on a wavelength of the diagnostic beam and output a test result of the specimen based on the reaction data.

2. The specimen testing device of claim 1, wherein each chamber is provided with a light source incident portion that laterally protrudes, and
the optical part causes the diagnostic beam to enter the light source incident portion.

3. The specimen testing device of claim 1, wherein the optical part includes:
a transfer unit installed in parallel with the specimen reaction part; and
a plurality of optical units coupled to the transfer unit to irradiate each chamber with the diagnostic beam and acquire the optical signal received for each chamber while sequentially moving along the arrangement direction of the chambers.

4. The specimen testing device of claim 3, wherein the plurality of optical units include:
a first optical unit that emits a first diagnostic beam; and
a second optical unit that is disposed in parallel with the first optical unit and emits a second diagnostic beam, and
the first diagnostic beam and the second diagnostic beam have different fluorescence wavelengths.

5. The specimen testing device of claim 4, wherein the fluorescence wavelengths include at least one selected from the group consisting of FAM, HEX, ROX, or Cy5,
the FAM fluorescence wavelength has an emission wavelength within a range of 510 nm to 530 nm,
the HEX fluorescence wavelength has an emission wavelength within a range of 550 nm to 580 nm,
the ROX fluorescence wavelength has an emission wavelength within a range of 595 nm to 635 nm, and
the Cy5 fluorescence wavelength has an emission wavelength within a range of 655 nm to 675 nm.

6. The specimen testing device of claim 4, wherein the first optical unit irradiates each chamber with the first diagnostic beam and acquires the optical signal for the first diagnostic beam for each chamber while sequentially moving along the arrangement direction of the plurality of chambers, and
the second optical unit irradiates each chamber with the second diagnostic beam and acquires the optical signal for the second diagnostic beam for each chamber while sequentially moving along the arrangement direction of the plurality of chambers.

7. The specimen testing device of claim 4, wherein the plurality of optical units further include a third optical unit that is disposed in parallel with the second optical unit along the arrangement direction of the chambers and emits a third diagnostic beam, and
the third diagnostic beam has a different wavelength from the first diagnostic beam and the second diagnostic beam.

8. The specimen testing device of claim 7, wherein the third optical unit irradiates each chamber with the third diagnostic beam and acquires the optical signal for the third diagnostic beam for each chamber while sequentially moving along the arrangement direction of the plurality of chambers.

9. The specimen testing device of claim 7, wherein the plurality of optical units further include a fourth optical unit that is disposed in parallel with the third optical unit in the arrangement direction of the chambers and emits a fourth diagnostic beam, and
the fourth diagnostic beam has a different wavelength from the first diagnostic beam to the third diagnostic beam.

10. The specimen testing device of claim 9, wherein the fourth optical unit irradiates each chamber with the fourth diagnostic beam and acquires the optical signal for the fourth diagnostic beam for each chamber while sequentially moving along the arrangement direction of the plurality of chambers.

11. The specimen testing device of claim 3, wherein the transfer unit includes:
a transfer rail installed in parallel with the specimen reaction part in the arrangement direction of the plurality of chambers;
a transfer belt installed in parallel with the transfer rail and spaced apart from the specimen reaction part;
a drive unit including a pair of drive pulleys installed at both ends of the transfer belt and a drive motor that provides a rotational force to one of the drive pulleys;
a transfer member having an upper end coupled to the plurality of optical units and a lower end coupled to the transfer belt, wherein the lower end is coupled to the transfer rail to be movable along the transfer rail when the transfer belt operates; and
a chain member installed in parallel with and spaced apart from the transfer belt with the transfer rail interposed therebetween, and coupled to the transfer member to guide movement of the transfer member.

12. The specimen testing device of claim 1, wherein the specimen reaction part includes:
a container mounting portion into which a vial container containing a lysis solution mixed with the specimen is inserted;
a reagent reaction portion in which the plurality of chambers are arranged in a row and spaced apart from each other;
a lysis solution distribution portion inserted into the vial container by passing through a lower end of the vial container to receive the lysis solution and distribute the lysis solution to each chamber; and
a gasket member stacked between the lysis solution distribution portion and the reagent reaction portion to cover each chamber, wherein the gasket member is provided so that each guide pin for guiding flow of the lysis solution in the chamber passes therethrough and air is allowed to flow into the chamber.

13. The specimen testing device of claim 12, further comprising:
a heating part that operates below the reagent reaction portion to provide heat to each chamber so that each chamber is isothermally maintained at a preset temperature; and
a heat dissipation part coupled to the heating part to dissipate heat from the heating part.

14. The specimen testing device of claim 12, wherein the lysis solution distribution portion is provided with:
a distribution plate provided to correspond to the reagent reaction portion and having an inflow hole vertically passing therethrough in a center thereof;
an inflow portion provided to protrude vertically upward from the distribution plate on the same axis as the inflow hole to be insertable into the vial container;
a channel portion provided with a reference channel connected to the inflow hole on a lower surface of the distribution plate and branch channels branching off from the reference channel into a plurality of branches to guide flow of the lysis solution flowing into the inflow hole;
a plurality of guide pins provided to protrude vertically downward from the distribution plate at ends of the respective branch channels to correspond to the plurality of chambers; and
a plurality of air holes provided to vertically pass through the distribution plate and arranged to correspond to the respective guide pins, and
the lysis solution distribution portion is stacked and coupled to the reagent reaction portion so that each guide pin is inserted into the corresponding chamber and each air hole is connected to the corresponding chamber.

15. The specimen testing device of claim 14, wherein the guide pin is provided with a pin groove along a longitudinal direction of the guide pin in one surface connected to the branch channel to guide flow of the lysis solution flowing through the branch channel.

16. The specimen testing device of claim 14, wherein each air hole is provided to be spaced apart from an end of the branch channel with the corresponding guide pin interposed therebetween on an opposite side of one surface where the pin groove is provided.

17. The specimen testing device of claim 14, wherein the plurality of branch channels branch off from the reference channel to be bilaterally symmetrical with respect to the inflow hole and are provided to be curved at a predetermined curvature in a direction away from the inflow hole.

18. The specimen testing device of claim 14, wherein the distribution plate is provided with a plurality of air grooves having a diameter larger than a diameter of each air hole and arranged to correspond to the respective air holes, and
the specimen testing device further comprises a filter member inserted into each air groove and provided with a plurality of filter protrusions covering each air hole.
